Europäisches Patentamt

European Patent Office

Office européen des brevets

(11) Publication number: **0 385 312**
**A2**

## EUROPEAN PATENT APPLICATION

(21) Application number: **90103577.4**

(51) Int. Cl.⁵: **A61K 7/15**

(22) Date of filing: **23.02.90**

(30) Priority: **28.02.89 JP 47687/89**

(43) Date of publication of application:
**05.09.90 Bulletin 90/36**

(84) Designated Contracting States:
**AT CH DE ES FR GB LI NL**

(71) Applicant: **KAO CORPORATION**
**14-10, Nihonbashi Kayabacho 1-chome**
**Chuo-ku Tokyo(JP)**

(72) Inventor: **Nozaki, Toshio**
**3-28, Nakashizu, Sakura-shi**
**Chiba-ken(JP)**
Inventor: **Yamamoto, Hiromi**
**4-9-2, Oojidai, Sakura-shi**
**Chiba-ken(JP)**
Inventor: **Minowa, Hiroki**
**1-3, Asahigaoka-cho**
**Chiba-shi, Chiba-ken(JP)**

(74) Representative: **Patentanwälte Dipl.-Ing. A.**
**Grünecker, Dr.-Ing. H. Kinkeldey, Dr.-Ing. W.**
**Stockmair,**
**Dr. rer. nat. K. Schumann, Dipl.-Ing. P.H.**
**Jakob, Dr. rer. nat. G. Bezold**
**Maximilianstrasse 58**
**D-8000 München 22(DE)**

(54) **Preshave cosmetic preparation.**

(57) The preshave cosmetic preparation for an electric shaver according to the present invention comprises 0.1 to 10% by weight of a globular powder having a particle size of 1 to 50 um and a true specific gravity of 1.5 or less and at least 50% by weight of a volatile carrier.

EP 0 385 312 A2

## PRESHAVE COSMETIC PREPARATION

## BACKGROUND OF THE INVENTION

[Field of the Invention]

The present invention relates to a preshave cosmetic preparation for an electric shaver which is applied to the surface of the skin when beards are shaven with an electric shaver.

[Description of the Prior Art]

Recently, a marked increase is observed in the demand for an electric shaver due to the convenience and speediness of the same. When beards are shaven with a safety razor, generally, a soap liquid, a shaving foam, a shaving cream or the like is applied to the surface of the skin prior to shaving. However, when an electric shaver is used, generally, nothing is applied to the surface of the skin in order not to lose the convenience and speediness thereof.

However, in recent years, when shaving is conducted with an electric shaver as well, there is a trend for applying a preshave agent for an electric shaver prior to shaving for the purpose of improving the feeling on the surface of the skin and the shaving effect.

Examples of the preshave agent for an electric shaver include a powdery or stick-form agent comprising as a principal component calcium carbonate, talc, zinc stearate, mangesium carbonate, mica, etc. and a lotion in which an oil, such as an ester, is dissolved.

Moreover, U.S. Patent No. 3,734,858 discloses a preshave agent comprising a water absorbing powder, such as talc, a lower alcohol and an activator in combination with a spraying agent.

However, the above-mentioned powdery or stick-form preshave agent, although it is applied to adsorb oils and water secreted onto the surface of the skin so as to improve the feeling of an electric shaver on the skin surface, has drawbacks that uniform application to the surface of the skin cannot be conducted due to poor applicability and adhesion, and that whitish matter is left. On the other hand, when the above-mentioned lotion is used, sliminess is left due to oil components after application to the surface of the skin, so that it becomes difficult to attain refreshed feeling. Further, any marked effect cannot be recognized with respect to the use of these preshave agents.

Moreover, from any preshave agents containing an inorganic water absorbing powder, such as talc, even if it is in a liquid form, comfortable feeling could not be obtained in use because of the adhesion and poor sliding properties attributed to the powder.

## SUMMARY OF THE INVENTION

An object of the present invention is to provide a preshave cosmetic preparation for an electric shaver which improves shaving efficiency, can be uniformly applied to the skin and imparts comfortable feeling on the skin during and after shaving, without detriment to the convenience and speediness of the electric shaver.

The present inventors have made extensive and intensive studies and, as a result, have found that a preshave cosmetic preparation which attains the above-mentioned objective can be obtained by incorporating into a volatile carrier a predetermined amount of a powder having a specific particle size and morphology and having a true specific gravity of 1.5 or less.

The present invention has been made based on the above-mentioned finding. According to the present invention, there is provided a preshave cosmetic preparation for an electric shaver, which comprises 0.1 to 10% by weight of a globular powder having a particle size of 1 to 50 $\mu$m and a true specific gravity of 1.5 or less and at least 50% by weight of a volatile carrier.

## DETAILED DESCRIPTION OF THE INVENTION

Preferred examples of the powder used in the present invention include a globular powder comprised of a material having a true specific gravity of up to 1.5, such as nylon, polystyrene, polyethylene, polypropylene, polytetrafluoroethylene, silicone resin, polyvinyl chloride, polymer of acrylic acid or acrylic acid ester, polymer of methacrylic acid or methacrylic acid ester, acrylic acid/styrene copolymer and styrene/divinylbenzene copolymer. These globular powders may be employed individually or more than two kinds of the globular powders may be employed together. When a plate-form powder having a true specific gravity of 2.0 or higher, such as talc, is used as the powder, the dispersibility in a liquid is poor and the lubrication is poor between the electric shaver and the skin surface, which makes it impossible to attain desirable shaving efficiency.

The above-mentioned globular powder generally has a particle size of from 1 to 50 $\mu$m, preferably from 3 to 20 $\mu$m. When the particle size is less than 1 $\mu$m, the lubricating property is poor. On the other hand, when the particle size exceeds 50 $\mu$m, harshness is felt on the skin.

The particle size of said globular powder is determined by an electron microphotograph.

The true specific gravity of the above-mentioned globular powder should be 1.5 or less, preferably 0.9 to 1.3. When the true specific gravity exceeds 1.5, the dispersibility in the volatile carrier is disadvantageously poor.

The content of the above-mentioned globular powder is 0.1 to 10% by weight, preferably 1 to 5% by weight. In light of the conventional application amount of preshave cosmetic preparations to the skin, when the content is less than 0.1% by weight, the effect desired in the present invention cannot be attained. On the other hand, when the content exceeds 10% by weight, whitish matter is left.

The type of the volatile carrier to be used in the present invention is not critical as long as use in cosmetics is acceptable. A volatile carrier which exhibits a vapor pressure of 60 mmHg at 300°C or below, especially 50°C or below, is preferred. Examples of such a volatile carrier include a cyclic dimethylpolysiloxane having about 3 to 7 silicon atoms, a linear dimethylpolysiloxane having about 3 to 9 silicon atoms, a straight-chain or branched hydrocarbon having about 6 to 16 carbon atoms and a lower alcohol having 1 to 4 carbon atoms. Especially preferably used are a lower alcohol, such as ethanol and methanol, a volatile dimenthylpolysiloxane and the like. These volatile carriers may be employed individually or more than two kinds of volatile carriers may be employed together.

The content of the above-mentioned volatile carrier is at least 50% by weight, preferably at least 70% by weight. When the content is less than 50% by weight, the drying characteristics of the preshave cosmetic preparation after application thereof is poor, which is detrimental to the convenience and speediness in use of the electric shaver.

An antiinflammatory drug, such as dipotassium glycyrrhitinate and glycyrrhetinic acid, an algefacient, such as menthol and camhor, an additive generally incorporated in cosmetic preparations, such as an oil agent and an emulsifying agent, a perfume, a colorant, etc. can be added as an auxiliary to the preshave cosmetic preparation of the present invention, as long as they do not adversely affect the desired effect of the present invention. The amount of the agent added, such as an antiinflammatory drug and an algefacient, is preferably about 0.05 to 3% by weight.

The preshave cosmetic preparation of the present invention can be prepared by mixing the above-mentioned globular powder with the above-mentioned volatile carrier, optionally together with the above-mentioned auxiliary, in the above-mentioned proportions according to conventional methods. Generally, the preparation is packed in a container to supply as a product. Examples of said container include bottle for liquid cosmetic preparation, roll-on type container and aerosol container, etc.

In the preshave cosmetic preparation of the present invention, when packed in a general container, the globular powder precipitates on the bottom of the container. Accordingly, it is preferred that after the container is shaken well before use for the purpose of uniformly dispersing the powder, an appropriate amount of the preparation be applied to the surface of the skin.

According to the cosmetic preparation for an electric shaver of the present invention, it is possible to improve shaving efficiency, effect uniform application to skin and impart comfortable feeling on the skin during and after shaving without detrimental to the convenience and speediness of the electric shaver. That is, the preshave cosmetic preparation for an electric shaver of the present invention exhibits the following effects.

(1) The cosmetic preparation can be more easily uniformly applied to the skin than the conventional powdery or stick-form cosmetic preparations, because a powder is dispersed in a volatile carrier and then applied to the surface of the skin.

(2) The cosmetic preparation is completely free from sliminess attributed to oil components a exhibited in the case of the conventional lotion-type preparation, because a powder is used, and hence imparts refreshed feeling.

(3) The cosmetic preparation is not detrimental to the convenience and speediness of the electric shaver because its drying characteristics are excellent.

(4) The lubrication is excellent between the external blade (mesh blade) of the electric shaver and the surface of the skin because a globular powder is used, so that it is possible to improve shaving efficiency, minimize the number of unshaved beards and effect deep shaving.

(5) The feeling on the skin after shaving is refreshed and excellent due to the presence of a powder.

The present invention will now be described in more detail by way of the following Examples, Comparative Examples and Application Examples for showing the effect of the present invention.

Examples 1 to 4 and Comparative Examples 1 to 3

Preshave cosmetic preparations were prepared respectively as cosmetic preparations 1 to 4 of the present invention and comparative cosmetic preparations 1 to 3 according to the formulations indicated in Table 1.

Table 1

| unit: % by weight | | Ex. | | | | Comp. Ex. | | |
|---|---|---|---|---|---|---|---|---|
| | No. | 1 | 2 | 3 | 4 | 1 | 2 | 3 |
| powder | nylon powder ([*1]: 7µm) ([*2]: 1.0) | 3 | | | | 3 | | |
| | polystyrene powder ([*1]: 10 µm) ([*2]: 0.9) | | 4 | 2 | | | | |
| | polymethyl methacrylate powder ([*1]: 7µm) ([*2]: 1.2) | | | 2 | | | | |
| | styrene/divinylbenzene copolymer powder ([*1]: 6µm) ([*2]: 1.1) | | | | 3 | | | |
| | polyethylene powder ([*1]: 80µm) ([*2]: 0.9) | | | | | | 4 | |
| | talc ([*2]: 2.8) | | | | | | | 3 |
| volatile carrier | ethanol (99 v/v%) | 85 | 90 | 92 | 85 | 45 | 90 | 85 |
| glycyrrhetinic acid | | 0.1 | 0.1 | 0.1 | 0.1 | | 0.1 | 0.1 |
| dipotassium glycyrrhitinate | | | | | | 0.1 | | |
| perfume | | 0.5 | 0.5 | 0.5 | 0.5 | 0.5 | 0.5 | 0.5 |
| purified water | | 11.4 | 5.4 | 3.4 | 11.4 | 51.4 | 6.4 | 11.4 |
| Note : | | | | | | | | |

[*1] Average particle size
[*2] True specific gravity

Comparative Example 4

A commercially available preshaving lotion containing no powder and comprising an oil as a principal component was obtained, which was designated comparative preparation 4.

Application Example 1

For the cosmetic preparations 1 to 4 of the present invention and comparative cosmetic preparations 1 to 4, the relative surface friction between an artificial skin (ALLOASK mfd. by Kotaikasei Kogyo K.K. regenerated with water) and an external blade (mesh blade) of an electric shaver was measured according to the following method (surface property tester: model 14 manufactured by HEIDON). The results are shown in Table 2.

(Measuring Method)

0.2 g of preshave cosmetic preparation was applied to ALLOASK of 30 x 60 mm, and the relative surface friction (the surface friction exhibited when the preshave cosmetic preparation was not applied was presumed to be 100) was measured 30 sec after the application.

Table 2

|  | Cosmetic preparation of the present invention | | | | Comparative cosmetic preparation | | | |
| --- | --- | --- | --- | --- | --- | --- | --- | --- |
|  | 1 | 2 | 3 | 4 | 1 | 2 | 3 | 4 |
| relative surface friction | 52 | 56 | 57 | 58 | 98 | 76 | 72 | 85 |

From the results shown in the above Table 2, it is apparent that the cosmetic preparation 1 to 4 of the present invention are both superior to the comparative cosmetic preparations 1 to 4 in the lubrication between an external blade (mess blade) of an electric shaver and skin surface.

Application Example 2

For the cosmetic preparations 1 to 4 of the present invention and comparative cosmetic preparations 1 to 4, the number of unshaved beards and the weight of beards shaved off were measured according to the following methods. The results are shown in Table 3.

(Measuring Method)

Application of each preshave cosmetic preparation and shaving by means of an electric shaver were conducted by a male once a day. The number of unshaved beards was counted by visual observation. The weight of shaved-off beards was measured by collecting the shaved-off beards and separating them in a solution of chloroform/ether = 7/3 (volume ratio), drying the separated shaved beards, and measuring the weight.

Table 3

|  | Cosmetic preparation of the present invention | | | | Comparative cosmetic preparation | | | |
| --- | --- | --- | --- | --- | --- | --- | --- | --- |
|  | 1 | 2 | 3 | 4 | 1 | 2 | 3 | 4 |
| number of unshaved beards | 9 | 12 | 15 | 12 | 18 | 31 | 24 | 38 |
| weight of beards shaved off (mg/day) | 41 | 39 | 37 | 40 | 29 | 30 | 30 | 23 |

From the results shown in the above Table 3, it is apparent that the cosmetic preparation 1 to 4 of the present invention are both less in the number of unshaved beards and more in the weight of shaved-off beards, thereby attesting to excellent shaving effect, as compared with the comparative cosmetic preparations 1 to 4.

Application Example 3

For the cosmetic preparations 1 to 4 of the present invention and comparative cosmetic preparations 1 to 4, one-week application test was conducted by 50 males, and the shaving easiness and feeling on the skin after shaving were evaluated. The results are shown in Table 4 below.

Table 4

| unit: no of persons | | | | |
| --- | --- | --- | --- | --- |
| | | | shaving easiness | feeling after shaving |
| Cosmetic preparation of | 1 | good | 92 | 90 |
| | | not clear | 8 | 10 |
| | | bad | 0 | 0 |
| the present invention | 2 | good | 88 | 86 |
| | | not clear | 12 | 14 |
| | | bad | 0 | 0 |
| | 3 | good | 85 | 83 |
| | | not clear | 15 | 17 |
| | | bad | 0 | 0 |
| | 4 | good | 87 | 88 |
| | | not clear | 13 | 12 |
| | | bad | 0 | 0 |
| Comparative cosmetic preparation | 1 | good | 12 | 62 |
| | | not clear | 26 | 28 |
| | | bad | 62 | 10 |
| | 2 | good | 36 | 12 |
| | | not clear | 22 | 18 |
| | | bad | 42 | 70 |
| | 3 | good | 46 | 36 |
| | | not clear | 34 | 30 |
| | | bad | 20 | 34 |
| | 4 | good | 20 | 4 |
| | | not clear | 12 | 16 |
| | | bad | 68 | 80 |

From the results shown in the above Table 4, it is apparent that the cosmetic preparations 1 to 4 of the present invention are both superior to the comparative cosmetic preparations 1 to 4 in shaving easiness and feeling on the skin after shaving.

| Example 5 | |
|---|---|
| Silicon treatment nylon powder (average particle size : 5μm true specific gravity : 1.0) | 2 % by weight |
| 99 % ethanol<br>Glyecyrrhetinic acid<br>Perfume<br>Water | 80 % by weight<br>0.1% by weight<br>0.5% by weight<br>17.4% by weight |

Preshave cosmetic preparation was prepared as cosmetic preparation 5 of the present invention according to the above-mentioned formulation.

Further, the above-mentioned silicon treatment nylon powder was prepared mentioned below. That is, 10 parts by weight of dimethylpolysiloxane [SH 200 mfd. by Tore Silicon Co., Ltd.; 5000 cs (25°C)] and 100 parts by weight of nylon powder (SP-500 mfd. by Tore Co., Ltd.; average particle size: 5 μm) were mixed in Henschel mixer for five minutes at a room temperature. And then the mixture was treated by heating in a decompression drier under 5 Torr at 120°C for 24 hours.

The cosmetic preparation 5 of the present invention has such as excellent effects as less in the number of unshaved beards and good sliding property of an electric shaver to the skin.

## Claims

1. A preshave cosmetic preparation for an electric shaver, which comprises 0.1 to 10% by weight of a globular powder having a particle size of 1 to 50 um and a true specific gravity of 1.5 or less and at least 50% by weight of a volatile carrier.

2. A cosmetic preparation according to claim 1, wherein said globular powder comprises one or more materials selected from the group consisting of nylon, polystyrene, polyethylene, polypropylene, polytetrafluoroethylene, silicone resin, polyvinyl chloride, polymer of acrylic acid or acrylic acid ester, polymer of methacrylic acid or methacrylic acid ester, acrylic acid/styrene copolymer and styrene/divinylbenzene copolymer.

3. A cosmetic preparation according to claim 1, wherein said volatile carrier is a volatile material which exhibits a vapor pressure of 60 mmHg at 300°C or below.

4. A cosmetic preparation according to claim 3, wherein said volatile material is selected from the group consisting of a cyclic dimethylpolysiloxane having about 3 to 7 silicon atoms, a linear dimethylpolysiloxane having about 3 to 9 silicon atoms, a straight-chain or branched hydrocarbon having about 6 to 16 carbon atoms, and a lower alcohol having 1 to 4 carbon atoms.